# EUROPÄISCHE PATENTANMELDUNG

(11) **EP 2 238 887 A1**
(43) Veröffentlichungstag der Anmeldung: **13.10.2010**
(21) Anmeldenummer: 10159252.5
(22) Anmeldetag: 07.04.2010
(51) Int. Cl.: A61B 1/04, A61B 5/00

(54) **Vorrichtung zur Fluoreszenzdiagnose**

(30) Priorität: 08.04.2009 DE 102009018142
(71) Anmelder: Karl Storz GmbH & Co. KG, 78532 Tuttlingen (DE)
(72) Erfinder: Irion, Klaus-Martin, 78576, Emmingen-Liptingen (DE); Ehrhardt, André, 78573, Wurmlingen (DE); Solleder, Peter, 78464, Konstanz (DE)
(74) Vertreter: Witte, Weller & Partner

(57) **Zusammenfassung**

Eine Vorrichtung zur Fluoreszenzdiagnose weist ein Beleuchtungssystem (12) zum Beleuchten eines Zielgebiets (16), und ein Beobachtungssystem (14) zum Beobachten des Zielgebiets (16) auf, wobei das Beobachtungssystem (14) ein Beobachtungsinstrument (54) und eine Kamera (58) aufweist, wobei das Beleuchtungssystem (12) zwischen zumindest zwei Beleuchtungsmodi umschaltbar ist, und wobei das Beobachtungssystem (14) eine optische Anordnung (74) aufweist, die zumindest ein optisches Element (78, 80, 82) aufweist, das wahlweise in den Beobachtungsstrahlengang einbringbar oder aus diesem entfernbar ist, um die optischen Eigenschaften des Beobachtungssystems (14) zwischen zumindest zwei Beobachtungsmodi umzuschalten. Die optische Anordnung (74) des Beobachtungssystems (14) weist einen motorischen Antrieb (90) auf, um das zumindest eine optische Element (78, 80, 82) in den Beobachtungsstrahlengang einzubringen und aus diesem zu entfernen, und es ist eine Steuerungseinheit (92) vorhanden, die mit dem Beleuchtungssystem (12) und mit dem Beobachtungssystem (14) verbunden ist, und die den motorischen Antrieb (90) in Abhängigkeit von dem eingestellten Beleuchtungsmodus des Beleuchtungssystems (12) ansteuert.

## Beschreibung

Die Erfindung betrifft eine Vorrichtung zur Fluoreszenzdiagnose, mit einem Beleuchtungssystem zum Beleuchten eines Zielgebiets und mit einem Beobachtungssystem zum Beobachten des Zielgebiets, wobei das Beobachtungssystem ein Beobachtungsinstrument und eine Kamera aufweist, wobei das Beleuchtungssystem zwischen zumindest zwei Beleuchtungsmodi umschaltbar ist, und wobei das Beobachtungssystem eine optische Anordnung aufweist, die zumindest ein optisches Element aufweist, das wahlweise in den Beobachtungsstrahlengang einbringbar oder aus diesem entfernbar ist, um die optischen Eigenschaften des Beobachtungssystems zwischen zumindest zwei Beobachtungsmodi umzuschalten.

Eine solche Vorrichtung zur Fluoreszenzdiagnose ist allgemein durch seine Verwendung in der medizinischen Diagnostik bekannt.

Eine erfindungsgemäße Vorrichtung zur Fluoreszenzdiagnose der eingangs genannten Art kann zu medizinischen Diagnosezwecken, aber auch zu technischen Diagnosezwecken in industriellen oder wissenschaftlichen Anwendungen verwendet werden. Obwohl die Erfindung hiernach mit Bezug auf die medizinische Fluoreszenzdiagnose beschrieben wird, ist die Erfindung hierauf nicht beschränkt.

Die medizinische Fluoreszenzdiagnose dient zur Beurteilung des Zustands von biologischem Gewebe, insbesondere zur Tumorerkennung. Mit einer Vorrichtung zur Fluoreszenzdiagnose der eingangs genannten Art kann die Fluoreszenzdiagnose insbesondere in vivo durchgeführt werden.

Bei der medizinischen Fluoreszenzdiagnose wird einem Patienten zuvor ein Photosensibilisator verabreicht oder in das zu beobachtende Zielgebiet, beispielsweise ein Gewebeareal, das auf das Vorhandensein von malignem Gewebe untersucht werden soll, eingebracht, wobei anstelle des Photosensibilisators auch eine Vorstufe desselben verabreicht oder eingebracht werden kann. Der Photosensibilisator kann durch Beleuchtung mit Licht in einem Spektralbereich, in dem der Photosensibilisator absorbiert, zur Fluoreszenz angeregt werden. Das Beleuchtungssystem der Vorrichtung zur Fluoreszenzdiagnose weist entsprechend zumindest einen Beleuchtungsmodus auf, in dem das Beleuchtungssystem dem Zielgebiet Licht in einem für die Fluoreszenzanregung geeigneten Spektralbereich zuführt. Das Fluoreszenzlicht des so angeregten Photosensibilisators wird dann über das Beobachtungsinstrument der Kamera zugeführt, wobei das von der Kamera aufgenommene Fluoreszenzbild auf einem Monitor visuell dargestellt werden kann.

Da die Intensität der Fluoreszenz deutlich geringer ist als die Intensität des Fluoreszenzanregungslichts, weist das Beobachtungssystem einen Beobachtungsmodus auf, in dem in den Beobachtungsstrahlengang ein optisches Element, üblicherweise ein Spektralfilter eingebracht wird, der im Wesentlichen nur das längerwellige Fluoreszenzlicht durchlässt, während es das intensivere kurzwelligere Fluoreszenzanregungslicht im Wesentlichen nicht durchlässt oder zumindest so weit schwächt, dass ein kontrastreiches Fluoreszenzbild beobachtet werden kann.

Der vorstehend beschriebene Beleuchtungsmodus des Beleuchtungssystems und der zugehörige Beobachtungsmodus des Beobachtungssystems wird üblicherweise und auch in der nachfolgenden Beschreibung als "Fluoreszenzmodus" bezeichnet.

Derzeit existierende Vorrichtungen zur Fluoreszenzdiagnose können jedoch nicht nur in einem einzigen Fluoreszenzmodus betrieben werden, sondern in mehreren, beispielsweise in zwei Fluoreszenzmodi. So kann eine Fluoreszenzdiagnose nicht nur anhand der Fluoreszenz eines Photosensibilisators, sondern auch anhand der Fluoreszenz des Gewebes selbst, der sogenannten Autofluoreszenz, durchgeführt werden. Da der Spektralbereich des Fluoreszenzanregungslichts und der Spektralbereich des Fluoreszenzlichts bei der Autofluoreszenz von dem Spektralbereich des Fluoreszenzanregungsspektrums und dem Spektralbereich des Fluoreszenzlichts eines Photosensibilisators unterschiedlich ist, sind bekannte Vorrichtungen zur Fluoreszenzdiagnose so ausgelegt, dass das Beleuchtungssystem zwischen zumindest zwei Beleuchtungsmodi umschaltbar ist, und zwar einem ersten Beleuchtungsmodus für die Fluoreszenzdiagnose mittels eines Photosensibilisators und einem zweiten Beleuchtungsmodus für die Autofluoreszenzdiagnose.

Entsprechend weist auch das Beobachtungssystem solcher Vorrichtungen zur Fluoreszenzdiagnose zumindest zwei Beobachtungsmodi auf, und zwar einen ersten Beobachtungsmodus für Fluoreszenzdiagnose mittels eines Photosensibilisators und einem Beobachtungsmodus für die Autofluoreszenz. Für diesen Fall weist die optische Anordnung des Beobachtungssystems zumindest zwei optische Elemente auf, beispielsweise zwei Spektralfilter mit unterschiedlichen Transmissionscharakteristika, die in das jeweilige Fluoreszenzspektrum des Photosensibilisators und deren gewebeeigenem Fluoreszenzspektrums angepasst sind.

Im Sinne der vorliegenden Erfindung können die zumindest zwei Beleuchtungsmodi und die zumindest zwei Beobachtungsmodi somit zwei unterschiedliche Fluoreszenzmodi sein.

Die vorliegende Erfindung ist jedoch nicht auf den vorstehend beschriebenen Fall beschränkt. Für den diagnostizierenden Arzt ist es bspw. aus Gründen der besseren Orientierung im Zielgebiet, d.h. in einem beobachteten Gewebeareal, erwünscht, das Zielgebiet auch unter üblicher Weißlichtbeleuchtung beobachten zu können. Derzeit bekannte Vorrichtungen zur Fluoreszenzdiagnose weisen daher auch einen Beleuchtungsmodus des Beleuchtungssystems auf, in dem das Beleuchtungssystem das Zielgebiet mit Weißlicht, d.h. mit Licht des gesamten sichtbaren Spektralbereichs, ausleuchtet. Zu diesem Beleuchtungsmodus gehört entsprechend ein Beobachtungsmodus des Beobachtungssystems, mit dem ein möglichst natürliches farbgetreues Bild des Zielgebiets beobachtet werden kann. Dazu wird ein zuvor für den Fluoreszenzmodus im Beobachtungsstrahlengang eingebrachtes Spektralfilter aus dem Beobachtungsstrahlengang wieder entfernt. Dieser Beleuchtungs- und Beobachtungsmodus wird üblicherweise und in der nachfolgenden Beschreibung als Weißlichtmodus bezeichnet.

Im Sinne der vorliegenden Erfindung können die zumindest zwei Beleuchtungsmodi und die zumindest zwei Beobachtungsmodi somit auch einen einzigen Fluoreszenzmodus und einen Weißlichtmodus umfassen. Im Sinne der vorliegenden Erfindung können aber auch mehr als zwei Beleuchtungs- und Beobachtungsmodi vorgesehen sein, beispielsweise zwei unterschiedliche Fluoreszenzmodi und ein Weißlichtmodus.

Während bei den bekannten Vorrichtungen zur Fluoreszenzdiagnose das Umschalten zwischen den zumindest zwei Beleuchtungsmodi aufgrund einer elektronischen Kopplung der Kamera mit dem Beleuchtungssystem durch an der Kamera befindliche Bedienelemente durchgeführt werden kann, muss zwischen den zugehörigen Beobachtungsmodi von Hand umgeschaltet werden, d.h. das zumindest eine optische Element des Beobachtungssystems, bspw. ein Spektralfilter, wird bei den bekannten Vorrichtungen manuell in den Beobachtungsstrahlengang eingebracht und manuell aus diesem entfernt. Bei den bekannten Vorrichtungen zur Fluoreszenzdiagnose ist die entsprechende optische Anordnung des Beobachtungssystems mit dem zumindest einen einbringbaren und entfernbaren optischen Element in das Beobachtungsinstrument, beispielsweise ein Endoskop, integriert.

In DE 197 13 276 C2 ist ein solches Endoskop offenbart, bei dem eine optische Anordnung mit mehreren optischen Elementen zum Umschalten zwischen verschiedenen Beobachtungsmodi, die mit dem Endoskop ermöglicht werden, über einen von Hand bedienbaren Stellring lageverstellbar ist, wobei der Stellring zur Lageverstellung der optischen Anordnung über eine Magnetkupplung mit der optischen Anordnung zusammenwirkt.

Das manuelle Umschalten zwischen den Beobachtungsmodi des Beobachtungssystems hat jedoch Nachteile. Durch das manuelle Umschalten zwischen den verschiedenen Beobachtungsmodi des Beobachtungssystems ist nicht immer gewährleistet, dass der manuell eingestellte Beobachtungsmodus auch zu dem aktuellen Beleuchtungsmodus des Beleuchtungssystems passt, weil der Benutzer irrtümlicherweise ein nicht an den aktuellen Beleuchtungsmodus angepasstes optisches Element in den Beobachtungsstrahlengang einführt. Bei den bekannten Vorrichtungen zur Fluoreszenzdiagnose besteht somit das Problem von Fehlbedienungen. Darüber hinaus ist die Durchführung der Fluoreszenzdiagnose aufgrund der manuellen Umschaltung zwischen den Beobachtungsmodi zeitaufwändig und umständlich.

Der Erfindung liegt daher die Aufgabe zugrunde, eine Vorrichtung zur Fluoreszenzdiagnose der eingangs genannten Art dahingehend weiterzubilden, dass ihre Bedienung vereinfacht und weniger zeitaufwändig ist und Fehlbedienungen möglichst ausgeschlossen sind.

Erfindungsgemäß wird diese Aufgabe hinsichtlich der eingangs genannten Vorrichtung dadurch gelöst, dass die optische Anordnung des Beobachtungssystems einen motorischen Antrieb aufweist, um das zumindest eine optische Element in den Beobachtungsstrahlengang einzubringen oder aus diesem zu entfernen, und dass eine Steuerungseinheit vorhanden ist, die mit dem Beleuchtungssystem und dem Beobachtungssystem verbunden ist, und die den motorischen Antrieb in Abhängigkeit von dem eingestellten Beleuchtungsmodus des Beleuchtungssystems ansteuert.

Die erfindungsgemäße Vorrichtung zur Fluoreszenzdiagnose ermöglicht nicht nur eine einfache Bedienung der Umschaltung zwischen den zumindest zwei Beobachtungsmodi, sondern die Umschaltung zwischen den zumindest zwei Beobachtungsmodi des Beobachtungssystems erfolgt auch automatisch in Abhängigkeit des jeweils eingestellten Beleuchtungsmodus des Beleuchtungssystems, ohne dass der Benutzer den jeweiligen Beobachtungsmodus an den jeweils aktuellen Beleuchtungsmodus manuell anpassen muss. Dazu ist die optische Anordnung des Beobachtungssystems mit einem motorischen, beispielsweise elektromotorischen Antrieb versehen, der von der Steuerungseinheit in Abhängigkeit von dem eingestellten Beleuchtungsmodus des Beleuchtungssystems angesteuert wird. Wenn der Benutzer der erfindungsgemäßen Vorrichtung einen bestimmten Betriebsmodus der Vorrichtung, beispielsweise über einen Bedienungsknopf an der Kamera einstellt, stellt die Steuerungseinheit den entsprechenden Beleuchtungsmodus und den zugehörigen Beobachtungsmodus automatisch ein. Schaltet der Benutzer beispielsweise vom Weißlichtmodus in den Fluoreszenzmodus um, steuert die Steuerungseinheit den motorischen Antrieb der optischen Anordnung des Beobachtungssystems an, um das zumindest eine optische Element, beispielsweise ein für den Fluoreszenzmodus geeignetes Spektralfilter, in den Beobachtungsstrahlengang zu bringen. Beim Umschalten auf den Weißlichtmodus wird das optische Element automatisch wieder aus dem Beobachtungsstrahlengang entfernt. Die erfindungsgemäße Vorrichtung zur Fluoreszenzdiagnose ist wesentlich einfacher zu bedienen als die bekannten Vorrichtungen zur Fluoreszenzdiagnose, und Fehlbedienungen sind ausgeschlossen, d.h. es kann nicht vorkommen, dass ein Beobachtungsmodus eingestellt wird, der nicht zu dem aktuellen Beleuchtungsmodus passt.

Die erfindungsgemäße Vorrichtung weist vorzugsweise ein Endoskop oder ein Operations-Mikroskop auf.

Der motorische Antrieb kann ein elektromotorischer Antrieb sein, wobei sich auch andere Antriebsarten, wie magnetische, hydraulische, piezoelektrische und elektromagnetische Antriebe gut eignen.

Vorzugsweise steuert die Steuerungseinheit auch das Kamerasystem in Abhängigkeit des Beleuchtungsmodus an, um den entsprechenden Farbmodus in der Kamera einzustellen.

In einer bevorzugten Ausgestaltung steuert die Steuerungseinheit den motorischen Antrieb synchron mit der Änderung des Beleuchtungsmodus des Beleuchtungssystems an.

Der Vorteil dieser Maßnahme besteht darin, dass die Umschaltung zwischen den Beobachtungsmodi zeitgleich mit der Umschaltung zwischen den Beleuchtungsmodi erfolgt und somit die Kamera jederzeit das dem eingestellten Beleuchtungsmodus entsprechende Bild des Zielgebiets korrekt empfängt. Auch steuert die Steuerungseinheit vorzugsweise synchron mit der Änderung des Beleuchtungsmodus den entsprechenden Farbmodus des Kamerasystems an.

In einer weiteren bevorzugten Ausgestaltung ist die optische Anordnung des Beobachtungssystems in der Kamera angeordnet.

Hierbei ist nun von Vorteil, dass die Integration des motorischen Antriebs in die Kamera wesentlich weniger aufwändig ist als die Integration des motorischen Antriebs in das Beobachtungsinstrument. Das Beobachtungsinstrument, beispielweise ein Endoskop oder ein Mikroskop, ist üblicherweise ein stromloses Instrument. Bei einer Integration einer motorisch angetriebenen optischen Anordnung in das Beobachtungsinstrument, insbesondere in ein Endoskop, muss beachtet werden, dass die Autoklavierbarkeit eines solchen Beobachtungsinstruments, das in den Körper eines Patienten eingeführt wird, gewährleistet bleiben muss. Wenn ein Endoskop jedoch einen motorischen Antrieb enthält, werden bspw. elektrischen Kontakte zur Versorgung und Steuerung des motorischen Antriebs benötigt, die jedoch nicht den thermischen Belastungen einer Dampfsterilisation in einem Autoklaven standhalten. Die Integration eines motorischen Antriebs in die Kamera ist demgegenüber wesentlich kostengünstiger und einfacher zu realisieren, da die Kamera nicht immer in einem Autoklaven sterilisiert werden muss. In manchen medizinischen Anwendungen ist es ausreichend, dass die Kamera durch einen sterilen Überzug ausreichend steril gehalten wird, da die Kamera stets außerhalb des Körpers verwendet wird. Darüber hinaus besteht ein weiterer Vorteil dieser Maßnahme darin, dass mehrere Beobachtungsinstrumente bspw. Endoskope mit unterschiedlichen Blickrichtungen mit derselben Kamera verwendet werden können, und somit die motorisch angetriebene optische Anordnung nur einmal, nämlich in der Kamera, vorgesehen werden muss.

In diesem Zusammenhang ist es bevorzugt, wenn die optische Anordnung im Bereich eines Objektivsystems der Kamera angeordnet ist.

Im Bereich des Objektivsystems der Kamera lässt sich die optische Anordnung mit dem motorischen Antrieb besonders vorteilhaft integrieren, insbesondere besteht in diesem Bereich auch eine gute Zugänglichkeit für den Austausch der optischen Anordnung.

Im Fall, dass die zumindest zwei Beobachtungsmodi zumindest einen Fluoreszenzmodus umfassen, ist das zumindest eine optische Element ein Spektralfilter. Zum Umschalten zwischen dem Fluoreszenzmodus und beispielsweise einem Weißlichtmodus oder einem anderen Fluoreszenzmodus wird das Spektralfilter über den motorischen Antrieb in den Beobachtungsstrahlengang eingebracht oder aus diesem wieder entfernt.

In einer weiteren bevorzugten Ausgestaltung, die insbesondere in Zusammenhang mit der vorstehend genannten Ausgestaltung bevorzugt ist, ist die optische Anordnung im Wesentlichen im parallelen Strahlengang des Beobachtungssystems angeordnet.

Diese Maßnahme ist vorteilhaft, weil insbesondere Spektralfilter in Form von Interferenzfiltern im nicht parallelen Strahlengang ihre spektrale Transmissionscharakteristik gegenüber ihrer Spezifikation verändern.

In einer weiteren bevorzugten Ausgestaltung stellt die Steuerungseinheit in Abhängigkeit des Beleuchtungsmodus und /oder des Beobachtungsmodus Kameraparameter der Kamera ein.

Hierbei ist von Vorteil, dass nicht nur die Beobachtungsmodi und die Beleuchtungsmodi über die Steuerungseinheit miteinander synchronisiert werden, sondern auch die Kameraparameter an den jeweiligen Beobachtungsmodus bzw. Beleuchtungsmodus synchron angepasst werden. Solche Kameraparameter können bspw. ein Weißlichtabgleich im Weißlichtmodus, die Integrationszeit(en) des (der) Bildaufnehmer(s) der Kamera im Falle einer elektronischen Kamera, die elektronische Verstärkung des Videosignals im Falle einer elektronischen Kamera, Helligkeitsregelungen des Kamerabildes, spezielle Farbeinstellungen für den entsprechenden Farbmodus der Kamera, usw., sein.

In einer weiteren bevorzugten Ausgestaltung ist die Steuerungseinheit mit dem Beobachtungssystem und dem Beleuchtungssystem über ein Kommunikationssystem verbunden, das aus der Gruppe ausgewählt ist, die einen Feldbus, Ethernet, Bluetooth, WLAN, USB oder dergleichen aufweist.

Die vorstehend genannten Kommunikationssysteme sind für die erfindungsgemäße Vorrichtung zur Fluoreszenzdiagnose besonders vorteilhaft geeignet, da sie auch dazu geeignet sind, dass die Steuerungseinheit oder ein Muster die angeschlossenen Systeme (Beobachtungssystem, Beleuchtungssystem, Kamera) und insbesondere deren Betriebszustand erkennen kann.

In einer weiteren bevorzugten Ausgestaltung weist die optische Anordnung des Beobachtungssystems einen motorisch angetriebenen Optikwechsler auf, der das zumindest eine optische Element aufweist.

Der Optikwechsler ist vorzugsweise als Rad ausgebildet, das um eine Achse parallel zur optischen Achse drehbar ist.

Die Ausgestaltung der optischen Anordnung mit einem motorisch angetriebenen Optikwechsler, der vorzugsweise als Rad ausgebildet ist, ermöglicht eine vorteilhaft kompakte Bauweise der optischen Anordnung des Beobachtungssystems, die für eine Integration in die Kamera des Beobachtungssystems geeignet ist. Das Rad muss hierbei weder kreisförmig noch vollumfänglich ausgebildet sein.

Wie bereits oben erwähnt, umfassen die zumindest zwei Betriebsmodi des Beleuchtungssystems vorzugsweise einen Weißlichtmodus und zumindest einen Fluoreszenzanregungsmodus.

Die erfindungsgemäße Vorrichtung eignet sich insbesondere vorteilhaft für eine Ausgestaltung, bei der das Beleuchtungssystem neben dem Weißlichtmodus einen ersten Fluoreszenzanregungsmodus umfasst, bei dem die spektrale Charakteristik des Beleuchtungssystems an einen Photosensibilisator angepasst ist, und einen Fluoreszenzanregungsmodus, bei dem die spektrale Charakteristik des Beleuchtungssystems an die Autofluoreszenz angepasst ist. Es können jedoch durchaus auch weitere Beleuchtungsmodi für das Beleuchtungssystem vorgesehen sein, wobei die vorliegende Erfindung gerade dann vorteilhaft ist, wenn das Beleuchtungssystem zwischen mehr als zwei Betriebsmodi umschaltbar ist, da in einem solchen Fall die Nachteile einer manuellen Umschaltung der Beobachtungsmodi zunehmen bzw. umgekehrt die Vorteile der automatischen, mit der Umschaltung der Beleuchtungsmodi synchronisierten Umschaltung der Beobachtungsmodi zunehmen.

In einer weiteren bevorzugten Ausgestaltung weist der Wechsler eine Mehrzahl von optischen Elementen auf, die vorzugsweise mehrere Spektralfilter für mehrere Spektralbereiche aufweisen.

Diese Ausgestaltung ist insbesondere von Vorteil, wenn das Beleuchtungssystem mehr als zwei Beleuchtungsmodi, insbesondere zumindest zwei Fluoreszenzmodi aufweist.

Das Einbringen bzw. Entfernen der optischen Elemente der optischen Anordnung des Beobachtungssystems erfolgt in jedem Fall vorzugsweise in Synchronisation mit dem eingestellten Beleuchtungsmodus des Beleuchtungssystems und dem entsprechenden Farbmodus des Kamerasystems ohne manuelle Intervention des Benutzers automatisch.

In einer weiteren bevorzugten Ausgestaltung weist das Beleuchtungssystem eine zweite optische Anordnung auf, die zumindest ein optisches Element aufweist, das wahlweise in den Beleuchtungsstrahlengang einbringbar und aus diesem entfernbar ist, um zwischen den zumindest zwei Beleuchtungsmodi umzuschalten.

Diese Ausgestaltung hat den Vorteil, dass das Beleuchtungssystem als Lichtquelle eine Weißlichtquelle aufweisen kann, während die unterschiedlichen optischen Eigenschaften, beispielsweise die spektralen Charakteristiken des Beleuchtungssystems, zwischen denen in den verschiedenen Beleuchtungsmodi umschaltbar ist, durch bewegliche optische Elemente, beispielsweise durch Spektralfilter, realisiert werden. Es ist zwar auch im Rahmen der Erfindung möglich, dass die verschiedenen Beleuchtungsmodi des Beleuchtungssystems durch eine Mehrzahl von Lichtquellen, beispielsweise mit unterschiedlichen Emissionscharakteristiken realisiert werden, jedoch hat die vorstehend genannte Ausgestaltung den Vorteil, weniger aufwändig und damit kostengünstiger und außerdem einfacher modifizierbar zu sein.

In einer weiteren bevorzugten Ausgestaltung weist die optische Anordnung des Beobachtungssystems und/oder die zweite optische Anordnung des Beleuchtungssystems einen Sensor, insbesondere Positionssensor auf, der die aktuelle Konfiguration der optischen Anordnung des Beobachtungssystems und/oder die aktuelle Konfiguration der zweiten optischen Anordnung des Beleuchtungssystems erfasst.

Hierbei ist von Vorteil, dass durch Erfassung der aktuellen Konfiguration der optischen Anordnung des Beobachtungssystems und/oder des Beleuchtungssystems die Synchronität und korrekte Anpassung des jeweils eingestellten Beobachtungsmodus an den jeweils eingestellten Beleuchtungsmodus mit hoher Funktionssicherheit gewährleistet wird.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale und Vorteile nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel ist in der Zeichnung dargestellt und wird mit Bezug auf diese hiernach näher beschrieben. Es zeigen:
- Fig. 1: eine Vorrichtung zur Fluoreszenzdiagnose in einem Blockschaltbild; und
- Fig. 2: eine optische Anordnung eines Beobachtungssystems der Vorrichtung zur Fluoreszenzdiagnose in Fig. 1 in Alleinstellung, in einem gegenüber Fig. 1 vergrößerten Maßstab und in einer Ansicht in Richtung der optischen Achse der optischen Anordnung.

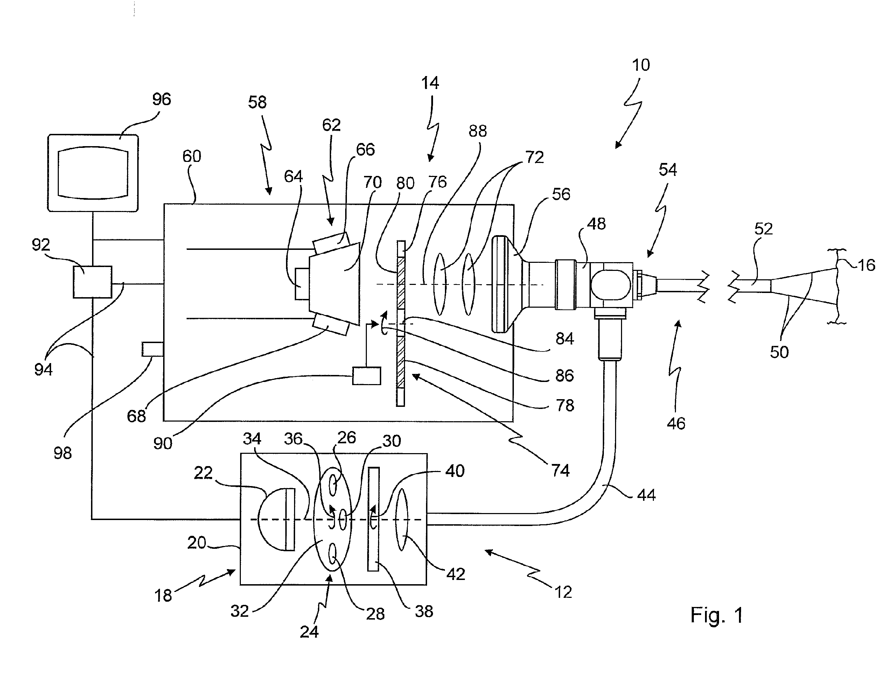

In Fig. 1 ist eine mit dem allgemeinen Bezugszeichen 10 versehene Vorrichtung zur Fluoreszenzdiagnose in einem Blockschaltbild dargestellt. Die Vorrichtung 10 wird ohne Beschränkung der Allgemeinheit nachfolgend anhand einer Verwendung zur medizinischen Fluoreszenzdiagnose beschrieben. Die Vorrichtung 10 kann jedoch auch zur technischen Fluoreszenzdiagnose für industrielle oder wissenschaftliche Zwecke eingesetzt werden.

Die Vorrichtung 10 weist allgemein ein Beleuchtungssystem 12 und ein Beobachtungssystems 14 auf.

Das Beleuchtungssystem 12 dient zum Beleuchten eines Zielgebiets 16, das ein Gewebeareal im menschlichen oder tierischen Körper sein kann, das Beobachtungssystem 14 zum Beobachten des Zielgebiets 16.

Das Beleuchtungssystem 12 weist eine Lichtquelle 18 auf. In einem Gehäuse 20 der Lichtquelle 18 ist eine Lampe 22 oder ein Lampensystem angeordnet, beispielsweise eine Xenon-Entladungslampe. Es können jedoch auch andere Weißlicht erzeugende Lampen oder Lampensysteme verwendet werden, wie beispielsweise Bogenentladungslampen, Glühlampen, LED-Lampensysteme, Laser- oder Laserdiodensysteme und dergleichen.

Die Lichtquelle 18 weist weiterhin eine optische Anordnung 24 auf, die zumindest ein optisches Element, hier drei optische Elemente 26, 28 und 30 aufweist. Zumindest zwei der optischen Elemente 26, 28 und 30 sind als Spektralfilter ausgebildet, wobei die Transmissionscharakteristika der zwei Spektralfilter unterschiedlich sind. Beispielsweise ist das optische Element 30 als Lichtdurchgangsöffnung ausgebildet, die die Eigenschaften, insbesondere das Spektrum des von der Lampe 22 emittierten Lichts nicht verändert.

Die optische Anordnung 24 des Beleuchtungssystems 12 weist einen Optikwechsler 32 auf, an dem die optischen Elemente 26, 28 und 30 angeordnet sind. Der Optikwechsler 32 ist, wie aus Fig. 1 ersichtlich, als Rad ausgebildet, der um eine Achse 34 gemäß einem Pfeil 36 drehbar ist. Der Optikwechsler 32 wird über einen nicht dargestellten, vorzugsweise elektromotorischen Antrieb angetrieben. Über den elektromotorischen Antrieb wird der Optikwechsler 32 so gedreht, dass jeweils eines der optischen Elemente 26, 28, 30 sich im Strahlengang des von der Lampe 22 emittierten Lichts befindet. Anstelle eines elektromotorischen Antriebs können auch magnetische, pneumatische, hydraulische, piezoelektrische und elektromagnetische Antriebe verwendet werden.

Wenn eines der als Filter ausgebildeten optischen Elemente 26, 28 in den Beleuchtungsstrahlengang des von der Lampe 22 emittierten Lichts gebracht wird, befindet sich das Beleuchtungssystem 12 jeweils in einem Fluoreszenzmodus, wobei das Beleuchtungssystem 12 in dem gezeigten Ausführungsbeispiel insgesamt zwei Fluoreszenzmodi mit unterschiedlichen spektralen Eigenschaften des Beleuchtungslichts aufweist. Ein erster Fluoreszenzmodus kann beispielsweise ein solcher sein, bei dem mit dem von der Lampe 22 emittierten Licht nach Durchgang durch das optische Element 26 Fluoreszenzanregungslicht in das Zielgebiet 16 abgestrahlt wird, um einen sich dort befindenden Photosensibilisator zur Fluoreszenz anzuregen. Ein zweiter Fluoreszenzmodus, der dadurch eingestellt wird, dass das optische Element 28 anstelle des optischen Elements 26 in den Beleuchtungsstrahlengang des von der Lampe 22 emittierten Lichts gebracht wird, kann beispielsweise zur Autofluoreszenzanregung von Gewebe im Zielgebiet 16 genutzt werden. In einem dritten Beleuchtungsmodus, der vorliegt, wenn das optische Element 30, das als Lichtdurchgangsöffnung für das von der Lampe 22 emittierte Licht ausgebildet ist, anstelle der optischen Elemente 26 und 28 im Beleuchtungsstrahlengang angeordnet ist, wird Weißlicht in das Zielgebiet 16 abgestrahlt (Weißlichtmodus).

In Lichtausbreitungsrichtung hinter der optischen Anordnung 24 ist ein Lichtabschwächer 38 angeordnet, der wahlweise in den Beleuchtungsstrahlengang eingeschwenkt und aus diesem wieder ausgeschwenkt werden kann, oder der, wie mit einem Pfeil 40 angedeutet ist, drehbar ist und mehrere Bereiche unterschiedlicher Lichtschwächung aufweist, um das Beleuchtungslicht zu dimmen.

Schließlich weist die Lichtquelle 18 ferner eine Kondensoroptik 42 auf, die das von der Lampe 22 emittierte Licht so bündelt, dass es in ein Lichtleitkabel 44 eingekoppelt wird, das die Lichtquelle 18 mit einem lichtzuführenden Instrument 46 verbindet. Über das Lichtleitkabel 44, das beispielsweise ein ungeordnetes Glasfaserbündel im Inneren enthält, wird das von der Lichtquelle 18 ausgehende Beleuchtungslicht in das lichtzuführende Instrument 46, das hier als Endoskop 48 mit einem integrierten Lichtleiter ausgebildet ist, in das Zielgebiet 16 (Lichtbündel 50) abgestrahlt. Das als lichtzuführendes Instrument 46 dienende Endoskop 48 weist einen langerstreckten Schaft 52 auf, der in den Körper eines Patienten in die Nähe des Zielgebiets 16 eingeführt wird.

Das Beobachtungssystem 14, mit dem das Zielgebiet 16 beobachtet werden kann, weist ein Beobachtungsinstrument 54 auf, das wie das lichtzuführende Instrument 46 durch das Endoskop 48 gebildet wird.

Es versteht sich, dass das lichtzuführende Instrument 46 und das Beobachtungsinstrument 54 anstatt durch ein Endoskop auch durch ein Mikroskop oder ein vergleichbares Instrument und auch durch getrennte Instrumente realisiert sein können. Das Endoskop 48 kann von jeder gebräuchlichen Bauart sein, wobei im vorliegenden Ausführungsbeispiel das Endoskop 48 ein Endoskop mit einem Okular 56 am proximalen Ende des Endoskops 48 ist.

An das Endoskop 48, genauer an das Okular 56 ist eine Kamera 58 als weiterer Bestandteil des Beobachtungssystems 14 angeschlossen.

In einem Gehäuse 60 der Kamera 58 ist eine Bildaufnehmereinheit 62 angeordnet, die drei Bildaufnehmer, beispielsweise CCD (Charge Coupled Device)-Chips, aufweist. Die drei Bildaufnehmer 64, 66, 68 sind in unterschiedlichen Spektralbereichen sensitiv, der Bildaufnehmer 64 beispielsweise im blauen Spektralbereich, der Bildaufnehmer 66 bspw. im roten Spektralbereich und der Bildaufnehmer 68 bspw. im grünen Spektralbereich. Die Verwendung einer Kamera mit drei Bildaufnehmern, die in unterschiedlichen Spektralbereichen sensitiv sind, hat den Vorteil einer verbesserten Trennung der Farbkanäle. Ein Beispiel einer solchen Kamera mit drei Bildaufnehmern wird von der Firma Karl Storz GmbH & Co. KG, Tuttlingen, Deutschland unter der Marke Tricam® SL-PDD vertrieben.

Beispielsweise wird mit dem Bildaufnehmer 64 vom Zielgebiet 16 rückgestreutes Fluoreszenzanregungslicht des Beleuchtungssystems 12 erfasst, mit dem Bildaufnehmerchip 66 wird Fluoreszenzlicht aus dem Zielgebiet 16 erfasst, und mit dem Bildaufnehmerchip 68 wird Autofluoreszenzlicht aus dem Zielgebiet 16 erfasst. Im Weißlichtmodus wird aus dem Zielgebiet 16 von dem Endoskop 48 übertragenes natürliches Farblicht von allen drei Bildaufnehmerchips 64, 66, 68 erfasst.

Die Kamera 58 kann des Weiteren auch noch einen vierten Bildaufnehmer (nicht dargestellt) aufweisen, der speziell im infraroten Spektralbereich sensitiv ist, so dass in Abhängigkeit von dem verwendeten Photosensibilisator auch Fluoreszenzlicht im infraroten Spektralbereich von der Kamera 58 aufgenommen und auf einem Bildschirm visuell, z.B. in Form einer Falschfarbendarstellung (da die Infrarot-Information nicht sichtbar ist), dargestellt werden kann.

Die Bildaufnehmereinheit 62 weist ferner ein Prisma 70 auf, das das vom Endoskop 48 übertragene Bild auf die Bildaufnehmer 64, 66, 68 verteilt.

In Lichtausbreitungsrichtung hinter dem Okular 56 des Endoskops 48 weist die Kamera 58 in dem Gehäuse 60 ein Objektivsystem 72 auf, die das vom Endoskop 48 übertragene Bild des Zielgebiets 16 auf die Bildaufnehmereinheit 62 abbildet.

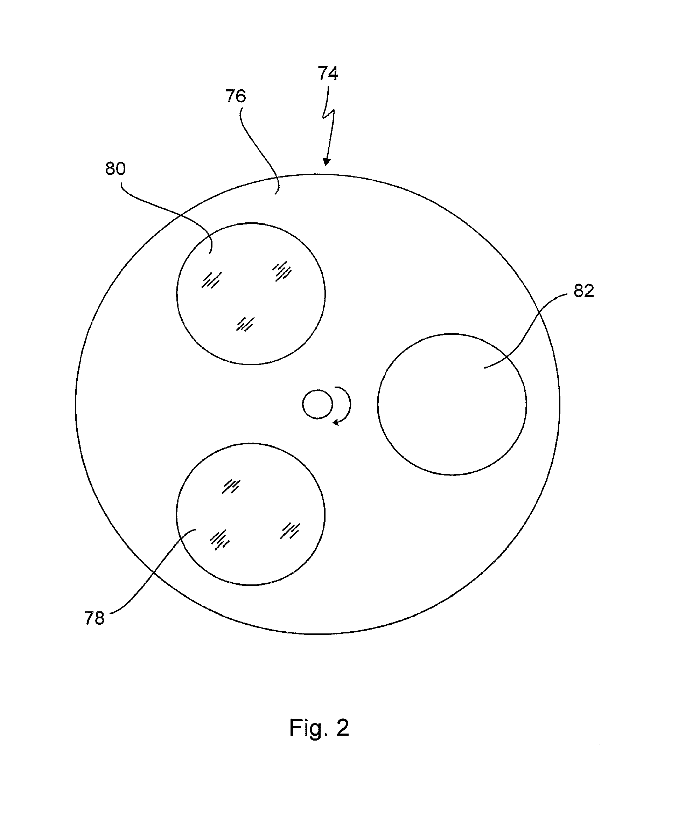

Die Kamera 58 weist weiterhin eine optische Anordnung 74 auf, die dazu dient, zwischen einer Mehrzahl von Beobachtungsmodi, die an den jeweils eingestellten Beleuchtungsmodus angepasst sind, umzuschalten. Die optische Anordnung 74 ist zusätzlich in Fig. 2 in Alleinstellung dargestellt.

Die optische Anordnung 74 weist einen Optikwechsler 76, hier einen Filterwechsler, auf, der eine Mehrzahl von optischen Elementen 78, 80 und 82 (Fig. 2) trägt.

Wie bereits mit Bezug auf das Beleuchtungssystem 12 oben beschrieben wurde, sind zwei der optischen Elemente, beispielsweise die optischen Elemente 78 und 80 als Spektralfilter mit zueinander unterschiedlichen spektralen Transmissionscharakteristika ausgebildet, während das optische Element 82 als Lichtdurchgangsöffnung ausgebildet ist, die die spektralen Eigenschaften des auf das optische Element 82 einfallende Licht nicht verändert.

Der Optik- bzw. Filterwechsler 76 ist als Rad ausgebildet, das um eine Achse 84 gemäß einem Pfeil 86 drehbar ist, wobei die Achse 84 parallel zur optischen Achse 88 des Beobachtungsstrahlengangs verläuft.

Die optische Anordnung 74, genauer gesagt die optischen Elemente 78, 80 und 82 sind in der Kamera 58 im Bereich des Objektivsystems 72 in Lichtausbreitungsrichtung vor der Bildaufnehmereinheit 62 angeordnet. Dabei ist die optische Anordnung 74 mit den optischen Elementen 78, 80, 82 insbesondere im Wesentlichen im parallelen Strahlengang des Beobachtungssystems 14 angeordnet, was insbesondere im Fall, dass zumindest eines der optischen Elemente 78, 80 und 82 ein Spektralfilter in Form eines Interferenzfilters ist, den Vorteil hat, dass die Transmissionscharakteristik dieses Spektralfilters nicht aufgrund eines divergenten Strahlengangs gegenüber der angegebenen Spezifikation des Spektralfilters verändert wird.

Die optische Anordnung 74 des Beobachtungssystems 14, das in die Kamera 58 integriert ist, weist einen elektromotorischen Antrieb 90 auf, der den Optikwechsler 76 bzw. Filterwechsler in Drehung um die Achse 84 versetzt, um jeweils eines der optischen Elemente 78, 80 bzw. 82 in den Beobachtungsstrahlengang zu bringen oder aus diesem wieder zu entfernen. Auf diese Weise werden die unterschiedlichen Beobachtungsmodi, von denen das Beobachtungssystem 14 im gezeigten Ausführungsbeispiel insgesamt drei aufweist, in Abhängigkeit von dem jeweils eingestellten Beleuchtungsmodus des Beleuchtungssystems 12 eingestellt. Das Einstellen des gewünschten Beobachtungsmodus erfolgt frei von einem manuellen Eingriff des Benutzers wie bei den herkömmlichen Vorrichtungen.

Anstelle des elektromotorischen Antriebs 90 können auch magnetische, pneumatische, hydraulische, piezoelektrische und elektromagnetische Antriebe verwendet werden.

Die elektrische Spannungsversorgung des elektromotorischen Antriebs kann über die bereits in der Kamera 58 vorhandene Spannungsversorgung (bspw. für die Bildaufnehmereinheit 62) erfolgen.

Um das jeweils zu dem eingestellten Beleuchtungsmodus des Beleuchtungssystems 12 passende optische Element 78, 80 oder 82 in den Beobachtungsstrahlengang zu bringen, ist eine Steuerungseinheit 92 vorhanden, beispielsweise eine Kamerasteuerungseinheit, die den elektromotorischen Antrieb 90 in Abhängigkeit des jeweils eingestellten oder aktuellen Beleuchtungsmodus des Beleuchtungssystems 12 ansteuert, damit der elektromotorische Antrieb 90 den Optikwechsler 76 so lageverstellt, dass das zum aktuellen Beleuchtungsmodus des Beleuchtungssystems 12 passende optische Element 78, 80 oder 82 in den Beobachtungsstrahlengang gebracht wird.

Dazu ist die Steuerungseinheit 92 mit dem Beleuchtungssystem 14, hier der Kamera 58 und dem Beleuchtungssystem 12, hier der Lichtquelle 18, verbunden.

Die Steuerungseinheit 92 steuert den elektromotorischen Antrieb 90 dabei insbesondere synchron mit der Änderung des Beleuchtungsmodus des Beleuchtungssystems 12 und den korrespondierenden Farbmodus der Kamera 58 an. Die Steuerungseinheit 92 weist dazu ein entsprechendes Steuerprogramm integriert auf.

Die Steuerungseinheit 92 ist mit der Kamera 58 und der Lichtquelle 80 über ein Kommunikationssystem 94 verbunden, das aus der Gruppe ausgewählt ist, die einen Feldbus, Ethernet, Bluetooth, WLAN, USB oder dergleichen aufweist. Im vorliegenden Ausführungsbeispiel ist das Kommunikationssystem94einSCB®-(STORZ Communication Bus) der Firma Karl Storz GmbH & Co. KG, Tuttlingen, Deutschland.

Zur Sicherstellung der korrekten Anpassung des jeweiligen Beobachtungsmodus an den aktuellen Beleuchtungsmodus weist die optische Anordnung 74 des Beobachtungssystems 14 und/oder die optische Anordnung 24 des Beleuchtungssystems 12 einen Positionssensor (nicht dargestellt) auf, der bzw. die die aktuelle Konfiguration der optischen Anordnung 74 des Beobachtungssystems 14 und/oder die aktuelle Konfiguration der optischen Anordnung 24 des Beleuchtungssystems 12 erfasst/
erfassen. Ein solcher Positionssensor kann beispielsweise die Drehstellung des jeweiligen Optikwechslers 76 bzw. 32 in Bezug auf eine Referenzdrehstellung erfassen.

Die Vorrichtung 10 weist ferner einen Monitor 96 auf, auf dem das von der Kamera 58 aufgenommene Bild des Zielgebiets 16 dargestellt werden kann.

Nachfolgend wird die Funktionsweise der Vorrichtung 10 beschrieben.

Über ein Bedienelement 98 stellt der Benutzer den gewünschten Betriebsmodus der Vorrichtung 10 ein. Der Benutzer kann dabei beispielsweise zwischen folgenden drei Betriebsmoden auswählen: Fluoreszenzmodus I (Fluoreszenz mit einem Photosensibilisator); Fluoreszenzmodus II (Autofluoreszenz); Weißlichtmodus.

Wählt der Benutzer beispielsweise Fluoreszenzmodus I, steuert die Steuerungseinheit 92 synchron das Beleuchtungssystem 12 und das Beobachtungssystems 14 an, um in der Lichtquelle 18 das zu dem Fluoreszenzmodus I gehörende optische Element 26 oder 28 in den Beleuchtungsstrahlengang zu bringen, falls zuvor eine andere Drehstellung des Optikwechslers 32 vorlag, und wählt den entsprechenden Farbmodus der Kamera an. Gleichzeitig steuert die Steuerungseinheit 92 den elektromotorischen Antrieb 90 an, der dann den Optikwechsler 76, falls erforderlich, in Drehung versetzt, um das zu dem Fluoreszenzmodus I passende optische Element 78 oder 80 in den Beobachtungsstrahlengang zu bringen. Möchte der Benutzer anschließend vom Fluoreszenzmodus I in den Fluoreszenzmodus II wechseln, betätigt er das Bedienelement 98 entsprechend, woraufhin die Steuerungseinheit 92 das Beleuchtungssystems 12 auf den entsprechenden Beleuchtungsmodus Fluoreszenzmodus II und das Beobachtungssystem 14 auf den entsprechenden Beobachtungsmodus Fluoreszenzmodus II einschließlich dem Farbmodus II in der Kamerasteuerung umschaltet. Dabei wird das zuvor im Beleuchtungsstrahlengang befindliche optische Element 26 oder 28 aus dem Beleuchtungsstrahlengang entfernt und das zu dem gewünschten Beleuchtungsmodus gehörige optische Element 26 oder 28 in den Beleuchtungsstrahlengang eingebracht, und ebenso wird im Beobachtungssystem 14 das zuvor im Beobachtungsstrahlengang befindliche optische Element 78 oder 80 aus dem Beobachtungsstrahlengang entfernt und das zum eingestellten Beleuchtungsmodus passende optische Element 78 oder 80 in den Beobachtungsstrahlengang gebracht.

Das Gleiche gilt, wenn der Benutzer vom Fluoreszenzmodus II in den Weißlichtmodus umschalten möchte, wobei dann die optischen Elemente 30 bzw. 82 in den jeweiligen Strahlengang gebracht werden und die Steuereinheit 92 die Farbwerte für den Standard-Weißlichtmodus der Kamera 58 liefert.

In Abhängigkeit des vom Benutzer gewählten Betriebsmodus der Vorrichtung 10 stellt die Steuerungseinheit 92 auch die entsprechenden Kameraparameter der Kamera ein, wie beispielsweise einen Weißlichtabgleich, die Integrationszeit der Bildaufnehmer 64, 66 und 68, die Helligkeit und dergleichen, so dass auch die Kameraeinstellung, insbesondere die Farbwerte automatisch und ohne manuellen Eingriff des Benutzers an den gewünschten und eingestellten Beleuchtungs- und Beobachtungsmodus der Vorrichtung 10 angepasst wird.

## Patentansprüche

1. Vorrichtung zur Fluoreszenzdiagnose, mit einem Beleuchtungssystem (12) zum Beleuchten eines Zielgebiets (16), und mit einem Beobachtungssystem (14) zum Beobachten des Zielgebiets (16), wobei das Beobachtungssystem (14) ein Beobachtungsinstrument (54) und eine Kamera (58) aufweist, wobei das Beleuchtungssystem (12) zwischen zumindest zwei Beleuchtungsmodi umschaltbar ist, und wobei das Beobachtungssystem (14) eine optische Anordnung (74) aufweist, die zumindest ein optisches Element (78, 80, 82) aufweist, das wahlweise in den Beobachtungsstrahlengang einbringbar oder aus diesem entfernbar ist, um die optischen Eigenschaften des Beobachtungssystems (14) zwischen zumindest zwei Beobachtungsmodi umzuschalten, **dadurch gekennzeichnet, dass** die optische Anordnung (74) des Beobachtungssystems (14) einen motorischen Antrieb aufweist, um das zumindest eine optische Element (78, 80, 82) in den Beobachtungsstrahlengang einzubringen oder aus diesem zu entfernen, und dass eine Steuerungseinheit (92) vorhanden ist, die mit dem Beleuchtungssystem (12) und mit dem Beobachtungssystem (14) verbunden ist, und die den motorischen Antrieb (90) in Abhängigkeit von dem eingestellten Beleuchtungsmodus des Beleuchtungssystems (12) ansteuert.

2. Vorrichtung nach Anspruch 1, **dadurch gekennzeichnet, dass** die Steuerungseinheit (92) in Abhängigkeit von dem Beleuchtungsmodus einen entsprechenden Farbmodus der Kamera (58) einstellt.

3. Vorrichtung nach Anspruch 1 oder 2, **dadurch gekennzeichnet, dass** die Steuerungseinheit (92) den motorischen Antrieb (90) und/oder den Farbmodus der Kamera (58) synchron mit der Änderung des Beleuchtungsmodus des Beleuchtungssystems (12) ansteuert.

4. Vorrichtung nach einem der Ansprüche 1 bis 3, **dadurch gekennzeichnet, dass** die optische Anordnung (74) des Beobachtungssystems (14) in der Kamera (58) angeordnet ist.

5. Vorrichtung nach Anspruch 4, **dadurch gekennzeichnet, dass** die optische Anordnung (74) im Bereich eines Objektivsystems (72) der Kamera (58) angeordnet ist.

6. Vorrichtung nach einem der Ansprüche 1 bis 5, **dadurch gekennzeichnet, dass** das zumindest eine optische Element (78, 80, 82) ein Spektralfilter ist.

7. Vorrichtung nach einem der Ansprüche 1 bis 6, **dadurch gekennzeichnet, dass** die optische Anordnung (74) im Wesentlichen im parallelen Strahlengang des Beobachtungssystems (14) angeordnet ist.

8. Vorrichtung nach einem der Ansprüche 1 bis 7, **dadurch gekennzeichnet, dass** die Steuerungseinheit (92) in Abhängigkeit des Beleuchtungsmodus und/oder des Beobachtungsmodus Kameraparameter der Kamera (58) einstellt.

9. Vorrichtung nach einem der Ansprüche 1 bis 8, **dadurch gekennzeichnet, dass** die Steuerungseinheit mit dem Beobachtungssystem (14) und dem Beleuchtungssystem (12) über ein Kommunikationssystem (94) verbunden ist, das aus der Gruppe ausgwählt ist, die einen Feldbus, Ethernet, Bluetooth, WLAN, USB oder dgl. aufweist.

10. Vorrichtung nach einem der Ansprüche 1 bis 9, **dadurch gekennzeichnet, dass** die optische Anordnung (74) des Beobachtungssystems (14) einen motorisch angetriebenen Optikwechsler (76), insbesondere Filterwechsler aufweist, der das zumindest eine optische Element (78, 80, 82) aufweist.

11. Vorrichtung nach Anspruch 10, **dadurch gekennzeichnet, dass** der Optikwechsler (76) als Rad ausgebildet ist, das um eine Achse (84) parallel zur optischen Achse (88) drehbar ist.

12. Vorrichtung nach einem der Ansprüche 1 bis 11, **dadurch gekennzeichnet, dass** die zumindest zwei Betriebsmodi des Beleuchtungssystems (12) einen Weißlichtmodus und zumindest einen Fluoreszenzanregungsmodus umfassen.

13. Vorrichtung nach einem der Ansprüche 10 bis 12, **dadurch gekennzeichnet, dass** der Optikwechsler (76) eine Mehrzahl von optischen Elementen (78, 80, 82) aufweist, die vorzugsweise mehrere Spektralfilter für mehrere Spektralbereiche aufweisen.

14. Vorrichtung nach einem der Ansprüche 1 bis 13, **dadurch gekennzeichnet, dass** das Beleuchtungssystem (12) eine zweite optische Anordnung (24) aufweist, die zumindest ein optisches Element (26, 28, 30) aufweist, das wahlweise in den Beleuchtungsstrahlengang einbringbar oder aus diesem entfernbar ist, um zwischen den zumindest zwei Beleuchtungsmodi umzuschalten.

15. Vorrichtung nach einem der Ansprüche 1 bis 14, **dadurch gekennzeichnet, dass** die optische Anordnung (74) des Beobachtungssystems (14) und/oder die zweite optische Anordnung (24) des Beleuchtungssystems (12) einen Sensor, insbesondere Positionssensor, aufweist, der die aktuelle Konfiguration der optischen Anordnung (74) des Beobachtungssystems (14) und/oder die aktuelle Konfiguration der zweiten optischen Anordnung (24) des Beleuchtungssystems (12) erfasst.
